# EUROPEAN PATENT APPLICATION

(11) **EP 3 949 989 A1**
(43) Date of publication of application: **09.02.2022**
(21) Application number: 20783563.8
(22) Date of filing: 17.02.2020
(51) Int. Cl.: A61K 39/395, A61P 9/14, A61P 25/00, A61P 37/06, C07K 16/28, C12N 15/13

(54) **ANTI IL-6 RECEPTOR ANTIBODY-CONTAINING INHIBITOR FOR INHIBITING DETERIORATION OF BBB FUNCTION**

(30) Priority: 29.03.2019 JP 2019068693
(71) Applicant: CHUGAI SEIYAKU KABUSHIKI KAISHA, Tokyo 115-8543 (JP)
(72) Inventor: TAKESHITA, Yukio, Ube-shi Yamaguchi 7558505 (JP); KANDA, Takashi, Ube-shi Yamaguchi 7558505 (JP); SERIZAWA, Kenichi, Gotemba-shi Shizuoka 4128513 (JP)
(74) Representative: Vossius & Partner Patentanwälte Rechtsanwälte mbB
(86) International application number: PCT/JP2020/005965
(87) International publication number: WO 2020/202839

(57) **Abstract**

Provided are suppressors of reduction of the blood-brain barrier (BBB) function, suppressors of the disruption of the tight junctions of the blood-brain barrier, suppressors of infiltration of leucocytes into the CNS, suppressors of permeation of IgGs in a patient's blood into the CNS, and therapeutic agents for neuromyelitis optica spectrum disorder, neuro-Behcet disease, neurosarcoidosis, central nervous system lupus (neuropsychiatric lupus), autoimmune encephalitis, or Vogt-Koyanagi-Harada disease which suppress reduction of blood-brain barrier function and/or restore reduced function of the blood-brain barrier, which contain an antibody containing a heavy chain variable region containing CDR1 having the sequence of SEQ ID NO: 1, CDR2 having the sequence of SEQ ID NO: 2, and CDR3 having the sequence of SEQ ID NO: 3, and a light chain variable region containing CDR1 having the sequence of SEQ ID NO: 4, CDR2 having the sequence of SEQ ID NO: 5, and CDR3 having the sequence of SEQ ID NO: 6.

SEQUENCE LISTING

<110>  CHUGAI SEIYAKU KABUSHIKI KAISHA

<120>  INHIBITOR OF BBB HYPOFUNCTION CONTAINING ANTI-IL-6 RECEPTOR ANTIBODY

<130>  C1-X1906P

<150>  JP 2019-068693
<151>  2019-03-29

<160>  10

<170>  PatentIn version 3.5

<210>  1
<211>  6
<212>  PRT
<213>  Artificial Sequence

<220>
<223>  An artificially synthesized sequence

<400>  1

His Asp His Ala Trp Ser
1               5


<210>  2
<211>  16
<212>  PRT
<213>  Artificial Sequence

<220>
<223>  An artificially synthesized sequence

<400>  2

Phe Ile Ser Tyr Ser Gly Ile Thr Asn Tyr Asn Pro Ser Leu Gln Gly
1               5                   10                  15


<210>  3
<211>  10
<212>  PRT
<213>  Artificial Sequence

<220>
<223>  An artificially synthesized sequence

<400>  3

Ser Leu Ala Arg Thr Thr Ala Met Asp Tyr
1               5                   10


<210>  4
<211>  11
<212>  PRT
<213>  Artificial Sequence

<220>

<223>  An artificially synthesized sequence

<400>  4

Gln Ala Ser Thr Asp Ile Ser Ser His Leu Asn
1               5                   10


<210>  5
<211>  7
<212>  PRT
<213>  Artificial Sequence

<220>
<223>  An artificially synthesized sequence

<400>  5

Tyr Gly Ser His Leu Leu Ser
1               5


<210>  6
<211>  9
<212>  PRT
<213>  Artificial Sequence

<220>
<223>  An artificially synthesized sequence

<400>  6

Gly Gln Gly Asn Arg Leu Pro Tyr Thr
1               5


<210>  7
<211>  107
<212>  PRT
<213>  Artificial Sequence

<220>
<223>  An artificially synthesized sequence

<400>  7

Asp Ile Gln Met Thr Gln Ser Pro Ser Ser Leu Ser Ala Ser Val Gly
1               5                   10                  15


Asp Ser Val Thr Ile Thr Cys Gln Ala Ser Thr Asp Ile Ser Ser His
            20                  25                  30


Leu Asn Trp Tyr Gln Gln Lys Pro Gly Lys Ala Pro Glu Leu Leu Ile
        35                  40                  45


Tyr Tyr Gly Ser His Leu Leu Ser Gly Val Pro Ser Arg Phe Ser Gly
    50                  55                  60


Ser Gly Ser Gly Thr Asp Phe Thr Phe Thr Ile Ser Ser Leu Glu Ala

65                  70                  75                  80

Glu Asp Ala Ala Thr Tyr Tyr Cys Gly Gln Gly Asn Arg Leu Pro Tyr
                85                  90                  95

Thr Phe Gly Gln Gly Thr Lys Val Glu Ile Glu
            100                 105

<210>  8
<211>  119
<212>  PRT
<213>  Artificial Sequence

<220>
<223>  An artificially synthesized sequence

<400>  8

Gln Val Gln Leu Gln Glu Ser Gly Pro Gly Leu Val Lys Pro Ser Glu
1               5                   10                  15

Thr Leu Ser Leu Thr Cys Ala Val Ser Gly His Ser Ile Ser His Asp
            20                  25                  30

His Ala Trp Ser Trp Val Arg Gln Pro Pro Gly Glu Gly Leu Glu Trp
        35                  40                  45

Ile Gly Phe Ile Ser Tyr Ser Gly Ile Thr Asn Tyr Asn Pro Ser Leu
    50                  55                  60

Gln Gly Arg Val Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Leu Ala Arg Thr Thr Ala Met Asp Tyr Trp Gly Glu Gly
            100                 105                 110

Thr Leu Val Thr Val Ser Ser
        115

<210>  9
<211>  214
<212>  PRT
<213>  Artificial Sequence

<220>
<223>  An artificially synthesized sequence

<400>  9

Asp Ile Gln Met Thr Gln Ser Pro Ser Ser Leu Ser Ala Ser Val Gly
1               5                   10                  15

Asp Ser Val Thr Ile Thr Cys Gln Ala Ser Thr Asp Ile Ser Ser His
            20                  25                  30

Leu Asn Trp Tyr Gln Gln Lys Pro Gly Lys Ala Pro Glu Leu Leu Ile
        35                  40                  45

Tyr Tyr Gly Ser His Leu Leu Ser Gly Val Pro Ser Arg Phe Ser Gly
    50                  55                  60

Ser Gly Ser Gly Thr Asp Phe Thr Phe Thr Ile Ser Ser Leu Glu Ala
65                  70                  75                  80

Glu Asp Ala Ala Thr Tyr Tyr Cys Gly Gln Gly Asn Arg Leu Pro Tyr
                85                  90                  95

Thr Phe Gly Gln Gly Thr Lys Val Glu Ile Glu Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  10
<211>  443
<212>  PRT
<213>  Artificial Sequence

<220>
<223>  An artificially synthesized sequence

<400>  10

Gln Val Gln Leu Gln Glu Ser Gly Pro Gly Leu Val Lys Pro Ser Glu
1               5                   10                  15

Thr Leu Ser Leu Thr Cys Ala Val Ser Gly His Ser Ile Ser His Asp
            20                  25                  30

His Ala Trp Ser Trp Val Arg Gln Pro Pro Gly Glu Gly Leu Glu Trp
        35                  40                  45

Ile Gly Phe Ile Ser Tyr Ser Gly Ile Thr Asn Tyr Asn Pro Ser Leu
    50                  55                  60

Gln Gly Arg Val Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Leu Ala Arg Thr Thr Ala Met Asp Tyr Trp Gly Glu Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Asn Phe Gly Thr Gln Thr Tyr Thr Cys Asn Val Asp His Lys Pro
        195                 200                 205

Ser Asn Thr Lys Val Asp Lys Thr Val Glu Arg Lys Ser Cys Val Glu
    210                 215                 220

Cys Pro Pro Cys Pro Ala Pro Pro Val Ala Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Phe Arg Val Val Ser Val Leu
    290                 295                 300

Thr Val Val His Gln Asp Trp Leu Asn Gly Lys Glu Tyr Lys Cys Lys
305                 310                 315                 320

Val Ser Asn Lys Gly Leu Pro Ala Pro Ile Glu Lys Thr Ile Ser Lys
                325                 330                 335

Thr Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Met Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Ala
            420                 425                 430

His Tyr Thr Gln Lys Ser Leu Ser Leu Ser Pro
        435                 440

## Description

### [Technical field]

The present invention relates to suppressors of the reduction of blood-brain barrier (BBB) function, suppressors of the disruption of the tight junctions of the blood-brain barrier, suppressors of leukocyte infiltration into the CNS, suppressors of the permeation of IgGs in a patient's blood into the CNS, as well as therapeutic agents for neuromyelitis optica spectrum disorder, neuro-Behcet disease, neurosarcoidosis, central nervous system lupus (neuropsychiatric lupus), autoimmune encephalitis, or Vogt-Koyanagi-Harada disease which suppress the reduction of the blood-brain barrier's function and/or restore the reduced function of the blood-brain barrier, comprising an antibody comprising a heavy chain variable region containing CDR1 having the sequence of SEQ ID NO: 1, CDR2 having the sequence of SEQ ID NO: 2, and CDR3 having the sequence of SEQ ID NO: 3, and a light chain variable region containing CDR1 having the sequence of SEQ ID NO: 4, CDR2 having the sequence of SEQ ID NO: 5, and CDR3 having the sequence of SEQ ID NO: 6.

### [Background Art]

The blood-brain barrier (BBB) prevents foreign substances or inflammatory cells from invading the central nervous system (CNS), and plays an important role in maintaining homeostasis of the central nervous system. Its basic structure is composed of three types of cells, which are vascular endothelial cells lined with a basement membrane, astrocytes, and pericytes. Endothelial cells form tight junctions, and pericytes line the vascular endothelial cells via the basement membrane. Moreover, astrocyte end-feet develop along the basement membrane to form glia limitans, and these tight junctions and glia limitans serve as physical barriers. In addition to physical barriers, BBB also has features such as strictly controlling the exchange of substances between the circulating blood and the central nervous system through various transporters and receptors that are expressed in the BBB. These features prevent foreign substances and inflammatory cells from invading the central nervous system and play an important role in maintaining homeostasis of the central nervous system. When this BBB function is disrupted for some reason, various disease-causing substances and inflammatory cells infiltrate the central nervous system, triggering an inflammatory reaction that seriously damages neurons.

Neuromyelitis optica-related disorder (Neuromyelitis optica spectrum disorder (NMOSD)) is a severe inflammatory demyelinating autoimmune disease that was initially called Devic's disease. In 2006, diagnostic criteria for NMO (involving at least optic neuritis and myelitis) were proposed by Wingerchuk et al., and in 2007, cases of optic neuritis or myelitis alone in addition to typical NMO cases came to be considered in the same category and referred to as NMOSD. Furthermore, the concept of NMOSD was proposed as a group of diseases in a broad sense in 2015, and now the diagnostic name NMOSD is widely and commonly used. It is clinically characterized by optic neuritis and/or transverse myelitis (NPL 1), and often causes severe dysfunctions, leading to various impairments such as visual impairment (blindness), movement impairment, and sensory impairment. NMOSD is a disease that repeats relapse and remission, and in severe relapses, it can lead to gait disturbance, complete paraplegia, or total sensory loss. In general, NMO pathology is not secondary progressive, and most of impairments are caused by severe, single, acute attacks.

SA237 is a modified IgG2 humanized anti-human IL-6 receptor neutralizing antibody designed to extend the half-life in plasma by modifying the amino acid sequence of tocilizumab, which is an IgG1 antibody. Compared to tocilizumab, SA237 has features such as 1) prolonged plasma half-life by pH-dependent IL-6 receptor binding, lowered antibody isoelectric point, and enhanced binding to FcRn under acidic conditions; and 2) reduced effector action such as ADCC/CDC by reduced Fcγ receptor binding ability and adoption of an IgG2 structure.

Clinical trials of SA237 have been conducted in patients with neuromyelitis optica spectrum disorder (NPLs 2 to 7).

It is known that the autoantibody anti-AQP4 antibody is involved in the pathogenesis of neuromyelitis optica spectrum disorder, and it has been reported that plasmablasts are the source of its production, that IL-6 promotes plasmablast survival and anti-AQP4 antibody production ability, and that these are inhibited by anti-IL-6 receptor antibodies (NPL 8). In addition, IL-6 has been reported to be involved in reduced BBB function and transfer of anti-AQP4 antibodies to the CNS because IL-6 concentration was high in the cerebrospinal fluid (CSF) of NMO patients and the permeability of BBB (blood-brain barrier) as assessed by the CSF/Serum Albumin ratio correlated with the IL-6 concentration in CSF (NPL 9). Furthermore, an evaluation using an *in vitro* BBB model in which endothelial cells and astrocytes were co-cultured revealed that NMO-IgG (IgG derived from NMO patients) acts on astrocytes to produce IL-6, and that IL-6 increases the permeability of endothelial cells and infiltration of inflammatory cells, and reduces BBB function. Furthermore, it has been reported that anti-IL-6 receptor antibodies suppress the enhancement of inflammatory cell infiltration by NMO-IgG (NPL 10).

However, it has not been known so far that SA237 suppresses the reduction of BBB function.

### [Citation List]

### [Non-Patent Literature]

[NPL 1] Lancet Neurol. 2007 Sep; 6(9):805-15
[NPL 2] https://clinicaltrials.gov/ct2/show/NCT02028884
[NPL 3] https://clinicaltrials.gov/ct2/show/study/NCT02073279?show_locs=Y#locn [NPL 4] https://www.clinicaltrialsregister.eu/ctr-search/search?query=SA-307JG
[NPL 5] https://www.clinicaltrialsregister.eu/ctr-search/trial/2015-005431-41/HR
[NPL 6] https://s3.amazonaws.com/gjcf-wp-uploads/wp-content/uploads/2016/05/16162202/12_12_14_Chugai_Webinar_PPT_Complete_Deck_FINAL. pdf
[NPL 7] EAN the home of neurology EPR3103 (https://ipp-ean18.netkey.at/index.php?p=recorddetail&rid=f16c1ff3-f5ec-4b71-8a99-7c39bdc90418&t)
[NPL 8] Chihara N et al., Proc Natl Acad Sci USA 2011; 108: 3701-3706
[NPL 9] Uchida T et al., Mult Scler 2017; 23: 1072-1084
[NPL 10] Takeshita Y et al., Neurol Neuroimmunol Neuroinflamm 2016; 4: e311

### [Summary of Invention]

### [Technical Problem]

The present invention was made in view of such circumstances. The present invention focuses on the fact that IL-6 and reduced blood-brain barrier function may be involved in neuromyelitis optica spectrum disorder, examines the effect of SA237 on the reduced function, and through this, aims at providing a new use of SA237, that is, its use as a suppressor of reduction of blood-brain barrier function and as a therapeutic agent for various diseases in which the concentration of IL-6 in the cerebrospinal fluid is high and the function of the blood-brain barrier is reduced.

### [Solution to Problem]

The present inventors conducted dedicated research in order to solve the above problem. As a result, they discovered that SA237 suppresses the reduction of blood-brain barrier function. In particular, they found that SA237 suppresses the disruption of tight junctions in the blood-brain barrier.

The present invention is based on such findings, and specifically includes the following:
[1] a suppressor of the reduction of blood-brain barrier function, comprising an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6;
[2] the suppressor according to [1], wherein the reduction of blood-brain barrier function is a disruption of a tight junction of the blood-brain barrier;
[3] the suppressor according to [1], wherein the reduction of blood-brain barrier function is infiltration of leukocytes into the CNS;
[4] the suppressor according to [1], wherein the reduction of blood-brain barrier function is permeation of IgGs in a patient's blood into the CNS;
[5] the suppressor according to any one of [1] to [4], which suppresses the reduction of blood-brain barrier function, the disruption of a tight junction of the blood-brain barrier, infiltration of leukocytes into the CNS, or permeation of IgGs in a patient's blood into the CNS in a patient with neuromyelitis optica spectrum disorder, neuro-Behcet disease, neurosarcoidosis, central nervous system lupus (neuropsychiatric lupus), autoimmune encephalitis, or Vogt-Koyanagi-Harada disease;
[6] a therapeutic agent for acute neuromyelitis optica spectrum disorder, wherein the therapeutic agent comprises an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6;
[7] an agent for restoring blood-brain barrier function, wherein the agent comprises an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6;
[8] a therapeutic agent for neuromyelitis optica spectrum disorder, neuro-Behcet disease, neurosarcoidosis, central nervous system lupus (neuropsychiatric lupus), autoimmune encephalitis, or Vogt-Koyanagi-Harada disease, wherein the therapeutic agent comprises an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6, and wherein the therapeutic agent suppresses the reduction of blood-brain barrier function and/or restores a reduced blood-brain barrier function;
[9] the therapeutic agent according to [8], wherein the reduction of blood brain barrier function is a disruption of a tight junction of the blood-brain barrier;
[10] the therapeutic agent according to [8], wherein the reduction of blood-brain barrier function is infiltration of leukocytes into the CNS;
[11] the therapeutic agent according to [8], wherein the reduction of blood-brain barrier function is permeation of IgGs in a patient's blood into the CNS;
[12] a therapeutic agent for neurosarcoidosis, central nervous system lupus (neuropsychiatric lupus), or Vogt-Koyanagi-Harada disease, wherein the therapeutic agent comprises an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6;
[13] the suppressor or therapeutic agent according to any one of [1] to [12], wherein the antibody comprises an antibody comprising the light chain variable region of SEQ ID NO: 7 and the heavy chain variable region of SEQ ID NO: 8; and
[14] the suppressor or therapeutic agent according to any one of [1] to [13], wherein the antibody comprises an antibody comprising the light chain of SEQ ID NO: 9 and the heavy chain of SEQ ID NO: 10.

The present invention also provides the following [1A] to [2D]:
[1A] a method of suppressing the reduction of blood-brain barrier function (for example, disruption of the tight junctions of the blood-brain barrier, leukocyte infiltration into the central nervous system (CNS), or permeation of IgGs in a patient's blood into the CNS) or a method of restoring a reduced function of the blood-brain barrier, wherein the method comprises administering an anti-IL-6 receptor antibody to a subject;
[1B] an anti-IL-6 receptor antibody for use in suppressing reduction of blood-brain barrier function (for example, disruption of the tight junctions of the blood-brain barrier, leukocyte infiltration into the central nervous system (CNS), or permeation of IgGs in a patient's blood into the CNS) or in restoring (promoting recovery of) a reduced blood-brain barrier function;
[1C] the use of an anti-IL-6 receptor antibody in the manufacture of a pharmaceutical composition for suppressing reduction of blood-brain barrier function (for example, disruption of tight junctions of the blood-brain barrier, leukocyte infiltration into the central nervous system (CNS), or permeation of IgGs in a patient's blood into the CNS) or for restoring a reduced function of the blood-brain barrier;
[1D] a pharmaceutical composition for suppressing reduction of blood-brain barrier function (for example, disruption of the tight junctions of the blood-brain barrier, leukocyte infiltration into the central nervous system (CNS), or permeation of IgGs in a patient's blood into the CNS) or for restoring a reduced blood-brain barrier function, wherein the pharmaceutical composition comprises an anti-IL-6 receptor antibody as an active ingredient;
[2A] a method of preventing or treating neuromyelitis optica spectrum disorder, neuro-Behcet disease, neurosarcoidosis, central nervous system lupus (neuropsychiatric lupus), autoimmune encephalitis, or Vogt-Koyanagi-Harada disease, wherein the method comprises administering an anti-IL-6 receptor antibody to a subject, whereby reduction of blood-brain barrier function is suppressed and/or reduced blood-brain barrier function is restored;
[2B] an anti-IL-6 receptor antibody for use in preventing or treating neuromyelitis optica spectrum disorder, neuro-Behcet disease, neurosarcoidosis, central nervous system lupus (neuropsychiatric lupus), autoimmune encephalitis, or Vogt-Koyanagi-Harada disease, wherein the anti-IL-6 receptor antibody suppresses reduction of blood-brain barrier function and/or restores a reduced blood-brain barrier function;
[2C] the use of an anti-IL-6 receptor antibody in the manufacture of a pharmaceutical composition for preventing or treating neuromyelitis optica spectrum disorder, neuro-Behcet disease, neurosarcoidosis, central nervous system lupus (neuropsychiatric lupus), autoimmune encephalitis, or Vogt-Koyanagi-Harada disease, wherein the antibody suppresses reduction of blood-brain barrier function and/or restores a reduced blood-brain barrier function; and
[2D] a pharmaceutical composition for preventing or treating neuromyelitis optica spectrum disorder, neuro-Behcet disease, neurosarcoidosis, central nervous system lupus (neuropsychiatric lupus), autoimmune encephalitis, or Vogt-Koyanagi-Harada disease, wherein the pharmaceutical composition comprises an anti-IL-6 receptor antibody as an active ingredient, and wherein the pharmaceutical composition suppresses reduction of blood-brain barrier function and/or restores a reduced blood-brain barrier function.

The anti-IL-6 receptor antibody described in [1A] to [2D] is an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6. It is preferably an antibody that comprises the light chain variable region of SEQ ID NO: 7 and the heavy chain variable region of SEQ ID NO: 8, and more preferably, an antibody that has the light chain of SEQ ID NO: 9 and the heavy chain of SEQ ID NO: 10.

### [Effects of the Invention]

The present invention has provided suppressors of reduction of blood-brain barrier functions comprising SA237.

### [Brief Description of Drawings]

Fig. 1 shows a schematic diagram of the *in vitro* BBB model used in the Examples.
Fig. 2 shows the results of evaluating the effect on the transepithelial/endothelial electrical resistance (TEER) values when IgGs purified from NMOSD patients' pooled sera and healthy subjects' pooled sera (NMOSD-IgG and Normal-IgG) were made to act from the astrocyte side and/or the endothelial cell side of the *in vitro* BBB model. The horizontal axis shows time, and the vertical axis shows transepithelial/endothelial electrical resistance (TEER) values.
Fig. 3 shows the results of evaluating the effect on TEER when NMOSD-IgG and SA237 were made to act simultaneously from the astrocyte side and the endothelial cell side of the *in vitro* BBB model.
Fig. 4 shows the results of evaluating the effect on TEER when NMOSD-IgG and SA237 were made to act simultaneously from the astrocyte side of the *in vitro* BBB model.
Fig. 5 shows the results of evaluating the effect on TEER when NMOSD-IgG and SA237 were made to act simultaneously from the endothelial cell side of the *in vitro* BBB model.
Fig. 6 shows the results of evaluating the effect on TEER when SA237 was made to act from the astrocyte side and endothelial cell side of the *in vitro* BBB model.
Fig. 7 shows the results of evaluating the effect on TEER when NMOSD-IgG was made to act from the astrocyte side of the *in vitro* BBB model, and 24 hours later, SA237 was made to act from the astrocyte side.
Fig. 8 shows the effect of SA237 on leukocyte infiltration induced by NMO-IgG in a leukocyte infiltration assay under a flow velocity load using a 3D Bioflux Flow Chamber. The number of total peripheral blood mononuclear cells (PBMCs), CD4⁺ cells, CD8⁺ cells, and CD19⁺ cells that have passed through the membrane is shown as a relative value to the control IgG-administered group. ^{∗} p <0.05, independent t-test (n = 6/group).

### [Description of Embodiments]

In one aspect, the present disclosure provides pharmaceutical compositions comprising an anti-IL-6 receptor antibody as an active ingredient. In one embodiment, the pharmaceutical compositions of the present invention can suppress the reduction of blood-brain barrier function and/or promote restoration of the reduced function by administration to a subject. Therefore, the pharmaceutical compositions of the present invention can also be expressed as suppressors of reduction of blood-brain barrier function, promoters of the restoration of blood-brain barrier function, or function-restoring agents. The pharmaceutical compositions of the present invention contain an amount (an effective amount) of an anti-IL-6 receptor antibody that is capable of suppressing reduction of blood-brain barrier function and/or promoting restoration of the reduced function in an administered subject.

Examples of the anti-IL-6 receptor antibody comprised in the pharmaceutical compositions of the present invention include an antibody comprising a heavy chain variable region comprising CDR1 having the sequence of SEQ ID NO: 1 (heavy chain CDR1 of SA237), CDR2 having the sequence of SEQ ID NO: 2 (heavy chain CDR2 of SA237), and CDR3 having the sequence of SEQ ID NO: 3 (heavy chain CDR3 of SA237), and a light chain variable region comprising CDR1 having the sequence of SEQ ID NO: 4 (light chain CDR1 of SA237), CDR2 having the sequence of SEQ ID NO: 5 (light chain CDR2 of SA237), and CDR3 having the sequence of SEQ ID NO: 6 (light chain CDR3 of SA237). The antibody is preferably an antibody comprising the heavy chain variable region of SEQ ID NO: 8 (heavy chain variable region of SA237) and the light chain variable region of SEQ ID NO: 7 (light chain variable region of SA237), and more preferably, an antibody comprising a heavy chain comprising the sequence of SEQ ID NO: 10 (heavy chain of SA237) and a light chain comprising the sequence of SEQ ID NO: 9 (light chain of SA237). SA237 is particularly preferable. As used herein, a "subject" is an individual whose blood-brain barrier function is reduced or who is at risk of having reduced blood-brain barrier function, and is preferably a human, but may also be a non-human mammal.

In the present specification, "a reduction of blood-brain barrier function" means a reduction in the barrier function of the blood-brain barrier (which can also be expressed as a disruption of the barrier function or a barrier dysfunction), and includes, for example, the disruption of the tight junctions of the blood-brain barrier, infiltration of foreign substances or inflammatory cells (e.g., leukocytes) into the CNS, and permeation of a patient's blood IgGs into the CNS. In one embodiment, the pharmaceutical composition of the present invention can suppress, by administration to a subject, one or more selected from the group consisting of disruption of tight junctions of the blood-brain barrier, infiltration of leukocytes into the CNS, and permeation of a patient's blood IgGs into the CNS. Therefore, the pharmaceutical composition of the present invention can be expressed as a suppressor of the disruption of the tight junctions of the blood-brain barrier, a suppressor of the infiltration of leukocytes into the CNS, or a suppressor of the permeation of a patient's blood IgGs into the CNS. Specific examples of leucocytes include lymphocytes (B cells, helper T cells, regulatory T cells, killer T cells, NK cells, NKT cells), granulocytes (neutrophils, eosinophils, basophils), monocytes, and such. Leukocytes are preferably peripheral blood mononuclear cells (PBMCs), more preferably CD4⁺ cells, CD8⁺ cells, and CD19⁺ cells, and particularly preferably, CD4⁺ cells and CD8⁺ cells.

The function of the blood-brain barrier can be evaluated using a known BBB model, for example, the *in vitro* BBB model prepared according to the method described in WO 2017/179375.

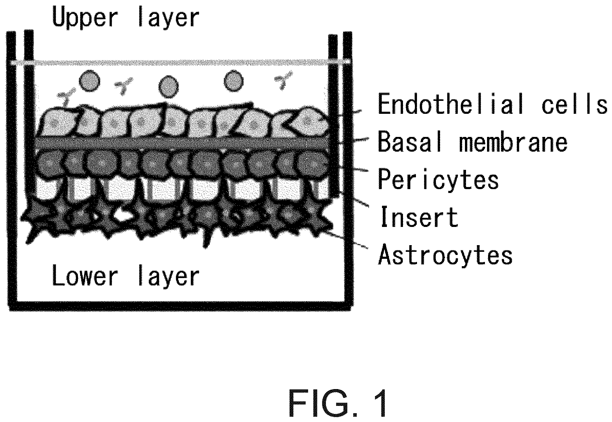

For example, as described in the examples of the present description, the tight junctions of the blood-brain barrier can be evaluated by measuring the change over time in transepithelial/endothelial electrical resistance (TEER) values when various molecules (e.g., IgGs purified from pooled sera of NMOSD patients and healthy subjects or SA237) are added from the endothelial cell side which is the upper layer of the *in vitro* BBB model shown in Fig. 1 (corresponding to the vascular lumen side), or from the astrocyte side which is the lower layer of the *in vitro* BBB model shown in Fig. 1 (corresponding to the central nervous system side), or from both sides. For example, when the decrease over time of TEER resulting from addition of IgGs derived from NMOSD patients (NMOSD-IgGs) is suppressed by application of the pharmaceutical composition of the present invention, the reduction of blood-brain barrier function is shown to be suppressed.

Moreover, the blood-brain barrier permeability to IgG can be evaluated by allowing NMOSD-IgGs to act from the endothelial cell side, which is the upper layer of the *in vitro* BBB model, then measuring the amount of NMOSD-IgGs on the astrocyte side, which is the lower layer. When the amount of IgGs on the lower layer side is reduced by the application of the pharmaceutical composition of the present invention, the reduction of blood-brain barrier function is shown to be suppressed.

A flow velocity load type BBB model (Takeshita Y et al., J Neurosci Methods 2014 Jul 30; 232: 165-172) can be used as another exemplary evaluation method. For example, by allowing inflammatory cells (for example, leukocytes) and such to flow in a chamber equipped with a membrane on which microvascular endothelial cells/pericytes/astrocytes have been co-cultured, then measuring foreign substances or inflammatory cells that infiltrated to the outside of the membrane in the presence of, for example, NMOSD-IgGs, the measurement can be used as an indicator of infiltration into the CNS.

As used herein, "suppression of the reduction of function" means that the reduction of blood-brain barrier function is decreased by administration of a pharmaceutical composition of the present invention; for example, it means that one or more selected from the disruption of the tight junctions of the blood-brain barrier, the infiltration of foreign substances or inflammatory cells (e.g., leucocytes) into the CNS, and the permeation of a patient's blood IgGs into the CNS are reduced. The suppression of the reduction of function does not necessarily have to be a complete prevention of the reduction of function, and it is sufficient that the reduction of function is decreased as compared to when the pharmaceutical composition of the present invention was not administered. Thus, "suppression of the reduction of function" can also be rephrased as an attenuation of the reduction of function. In the present specification, the degree of suppression of functional reduction is not limited, and the meaning of "suppression of the reduction of function" of the present invention includes the case where even a part of the reduction of blood-brain barrier function is decreased. In a specific embodiment, suppression of reduction of function can refer to a decrease of approximately 20%, 30%, 40%, 50%, 60%, 70%, 80%, 90%, or 95% of the reduction of the blood-brain barrier's function. "Suppression of the reduction of function" includes deterrence (prevention) of the reduction of function. As a more specific example, when the reduction of TEER, which is seen 120 hours after the addition of NMOSD-IgGs to both the endothelial cell side and the astrocyte side of an *in vitro* BBB model containing endothelial cells, basement membrane, pericytes, insert, and astrocytes, is suppressed by about 20%, 30%, 40%, 50%, 60%, 70%, 80%, 90%, or 95% by the addition of a pharmaceutical composition of the present invention to both the endothelial cell side and the astrocyte side at the same time as the NMOSD-IgGs, the reduction of blood-brain barrier function is shown to be suppressed.

Further, as used herein, "restoration of a function" means that a reduced function of the blood-brain barrier is improved by administration of a pharmaceutical composition of the present invention; for example, it indicates one or more selected from: recovery of disrupted tight junctions of the blood-brain barrier, reduction of the infiltration of foreign substances or inflammatory cells (e.g., leukocytes) into the CNS, and reduction of the permeation of the patient's blood IgGs into the CNS. The restoration of function does not necessarily have to be a complete restoration of the reduced function, and it is sufficient that the reduced function is improved as compared to when the pharmaceutical composition of the present invention was not administered. In the present specification, the degree of functional restoration is not limited, and the meaning of "restoration of a function" in the present invention includes the case where even part of the reduced blood-brain barrier function is improved. In a specific embodiment, restoration can refer to an improvement of approximately 20%, 30%, 40%, 50%, 60%, 70%, 80%, 90%, or 95% of a reduced blood-brain barrier function. As a more specific example, when TEER, which was reduced after (e.g., 24 hours after) the addition of NMOSD-IgGs to both the endothelial cell side and the astrocyte side of an *in vitro* BBB model containing endothelial cells, basement membrane, pericytes, insert, and astrocytes, increases by the addition 24 hours after the addition of NMOSD-IgGs of a pharmaceutical composition of the present invention by about 20%, 30%, 40%, 50%, 60%, 70%, 80%, 90%, or 95% (e.g., at 120 hours after the addition of the pharmaceutical composition of the present invention), the reduced blood-brain barrier function is shown to be restored.

In another embodiment, the pharmaceutical composition of the present invention, when administered to a subject, can suppress the reduction of the blood-brain barrier function and/or promote the recovery of the reduced function, and thus can prevent and/or treat diseases with high IL-6 concentration in the cerebrospinal fluid and involving a reduction of blood-brain barrier function. Examples of such diseases include, without limitation, neuromyelitis optica spectrum disorder (including acute neuromyelitis optica spectrum disorder), neuro-Behcet disease, neurosarcoidosis, central nervous system lupus (neuropsychiatric lupus), autoimmune encephalitis, and Vogt-Koyanagi-Harada disease. Therefore, the pharmaceutical compositions of the present invention can also be expressed as preventive and/or therapeutic agents for neuromyelitis optica spectrum disorder, neuro-Bechet's disease, neurosarcoidosis, central nervous system lupus (neuropsychiatric lupus), autoimmune encephalitis, or Vogt-Koyanagi-Harada disease, which suppress a reduction of blood-brain barrier function and/or restore a reduced blood-brain barrier function. The pharmaceutical compositions of the present invention are administered at a dose at which an anti-IL-6 receptor antibody, which is the active ingredient, can prevent and/or treat these diseases.

In another aspect, the present disclosure relates to a method for suppressing a reduction of blood-brain barrier function or a method for restoring a reduced blood-brain barrier function, which comprises administering to a subject an effective amount of an anti-IL-6 receptor antibody. Alternatively, the disclosure relates to a method for preventing or treating neuromyelitis optica spectrum disorder, neuro-Bechet's disease, neurosarcoidosis, central nervous system lupus (neuropsychiatric lupus), autoimmune encephalitis, or Vogt-Koyanagi-Harada disease by suppressing a reduction of blood-brain barrier function and/or restoring a reduced blood-brain barrier function, which comprises administering to a subject an effective amount of an anti-IL-6 receptor antibody. In one embodiment, the method further comprises administering to the subject at least one additional agent. The combined use of the additional agent with the anti-IL-6 receptor antibody encompasses combined administration (two or more agents contained in the same or separate formulation) and individual administration, and in the case of individual administration, the anti-IL-6 receptor antibody administration may be performed prior to, simultaneously, and/or subsequent to, administration of the additional agent. Examples of the additional agent include, but are not limited to, one or more agents selected from immunosuppressive agents and steroids.

In another aspect, the present disclosure relates to an anti-IL-6 receptor antibody for use in suppressing a reduction of blood-brain barrier function or in restoring (promoting recovery of) a reduced blood-brain barrier function. Alternatively, the present disclosure relates to an anti-IL-6 receptor antibody for use in preventing or treating neuromyelitis optica spectrum disorder, neuro-Behcet disease, neurosarcoidosis, central nervous system lupus (neuropsychiatric lupus), autoimmune encephalitis, or Vogt-Koyanagi-Harada disease, which suppresses a reduction of a blood-brain barrier function and/or restores a reduced blood-brain barrier function. Alternatively, the present disclosure relates to the use of an anti-IL-6 receptor antibody in suppressing a reduction of a blood-brain barrier function or in restoring a reduced blood-brain barrier function. Alternatively, the present disclosure relates to the use of an anti-IL-6 receptor antibody in the prevention or treatment of neuromyelitis optica spectrum disorder, neuro-Behcet disease, neurosarcoidosis, central nervous system lupus (neuropsychiatric lupus), autoimmune encephalitis, or Vogt-Koyanagi-Harada disease, wherein the antibody is an antibody that suppresses a reduction of blood-brain barrier function and/or restores a reduced blood-brain barrier function.

Alternatively, the present disclosure relates to the use of an anti-IL-6 receptor antibody in the manufacture of a pharmaceutical composition for suppressing a reduction of blood-brain barrier function or for restoring a reduced blood-brain barrier function. Alternatively, the present disclosure relates to the use of an anti-IL-6 receptor antibody in the manufacture of a pharmaceutical composition for preventing or treating neuromyelitis optica spectrum disorder, neuro-Behcet disease, neurosarcoidosis, central nervous system lupus (neuropsychiatric lupus), autoimmune encephalitis, or Vogt-Koyanagi-Harada disease, wherein the antibody is an antibody that suppresses a reduction of blood-brain barrier function and/or restores a reduced blood-brain barrier function. Alternatively, the present invention relates to a method of producing a pharmaceutical composition for suppressing a reduction of blood-brain barrier function or for restoring a reduced blood-brain barrier function, comprising mixing an anti-IL-6 receptor antibody with a pharmaceutically acceptable carrier. Alternatively, the present invention relates to a method of producing a pharmaceutical composition for preventing or treating neuromyelitis optica spectrum disorder, neuro-Behcet disease, neurosarcoidosis, central nervous system lupus (neuropsychiatric lupus), autoimmune encephalitis, or Vogt-Koyanagi-Harada disease, comprising mixing an anti-IL-6 receptor antibody with a pharmaceutically acceptable carrier, wherein the antibody is an antibody which suppresses a reduction of blood-brain barrier function and/or restores a reduced blood-brain barrier function. Such pharmaceutical compositions may include at least one additional agent in addition to the anti-IL-6 receptor antibody and pharmaceutically acceptable carrier.

In one embodiment, the pharmaceutical composition of the present invention may be formulated as a unit dosage form containing an effective amount of an anti-IL-6 receptor antibody. Herein, an "effective amount" refers to an amount at the necessary dose and over the necessary period effective for achieving the desired suppressive, function-restoring, therapeutic, or preventive result.

The dose of the pharmaceutical composition of the present invention can be appropriately set according to the condition of the subject of administration, the administration method (for example, number of administration times, frequency of administration, timing for administration, and administration route), and the like. In one embodiment, specific examples of the amount of anti-IL-6 receptor antibody contained in the pharmaceutical composition of the present invention per administration are, as doses per kg body weight, 2 to 20 mg (2 to 20 mg/kg), preferably 2 to 8 mg (2 to 8 mg/kg), and more preferably 8 mg (8 mg/kg), and are, as fixed doses, 50 to 800 mg/body, preferably 80 to 160 mg/body, and more preferably 120 mg/body; but are not limited thereto.

The subject of administration of the pharmaceutical composition of the present invention is a mammal. Mammals include, but are not limited to, domestic animals (for example, cows, sheep, cats, dogs, and horses), primates (for example, humans and non-human primates such as monkeys), rabbits, and rodents (for example, mice and rats). In a particular embodiment, the subject of administration of the pharmaceutical composition of the present invention is a human. In another embodiment, the subject of administration is a non-human mammal.

The pharmaceutical composition of the present invention comprises, as an active ingredient, an antibody against the IL-6 receptor.

The IL-6 receptor, which is a ligand-binding protein with a molecular weight of approximately 80 kD, binds to IL-6 to form an IL-6/IL-6 receptor complex. Then, binding of this complex to gp130, a membrane protein with a molecular weight of approximately 130 kD involved in non-ligand binding signal transduction, causes intracellular transduction of the biological activity of IL-6.

An anti-IL-6 receptor antibody used in the present invention can be obtained as either a polyclonal or monoclonal antibody using known methods. A monoclonal antibody derived from a mammal is particularly preferred for the anti-IL-6 receptor antibody used in the present invention. The monoclonal antibodies derived from a mammal include those produced by a hybridoma and those produced by a host transformed with an expression vector containing an antibody gene using genetic engineering methods. By binding to an IL-6 receptor, this antibody inhibits the binding of IL-6 to an IL-6 receptor, and blocks intracellular transduction of the IL-6 biological activity.

Examples of such an antibody include the MR16-1 antibody (Tamura, T. et al. Proc. Natl. Acad. Sci. USA (1993) 90, 11924-11928), PM-1 antibody (Hirata, Y. et al., J. Immunol. (1989) 143, 2900-2906), AUK12-20 antibody, AUK64-7 antibody, AUK146-15 antibody (International Patent Application Publication No. WO 92-19759), Sarilumab, Vobarilizumab, and BCD-089. Among them, the PM-1 antibody is listed as an example of a preferred monoclonal antibody against the human IL-6 receptor, and the MR16-1 antibody is listed an example of a preferred monoclonal antibody against the mouse IL-6 receptor.

Preferred examples of an "anti-IL-6 receptor antibody" of the present invention include tocilizumab which is a humanized anti-IL-6 receptor IgG1 antibody, and humanized anti-IL-6 receptor antibodies produced by modifying the variable and constant regions of tocilizumab. Specific examples include an antibody comprising a heavy chain variable region comprising CDR1 having the sequence of SEQ ID NO: 1 (heavy chain CDR1 of SA237), CDR2 having the sequence of SEQ ID NO: 2 (heavy chain CDR2 of SA237), and CDR3 having the sequence of SEQ ID NO: 3 (heavy chain CDR3 of SA237) and a light chain variable region comprising CDR1 having the sequence of SEQ ID NO: 4 (light chain CDR1 of SA237), CDR2 having the sequence of SEQ ID NO: 5 (light chain CDR2 of SA237), and CDR3 having the sequence of SEQ ID NO: 6 (light chain CDR3 of SA237). This antibody is preferably an antibody comprising the heavy chain variable region of SEQ ID NO: 8 (heavy chain variable region of SA237) and the light chain variable region of SEQ ID NO: 7 (light chain variable region of SA237), and more preferably, an antibody comprising a heavy chain comprising the sequence of SEQ ID NO: 10 (heavy chain of SA237) and a light chain comprising the sequence of SEQ ID NO: 9 (light chain of SA237). SA237 is particularly preferable.

These antibodies can be obtained according to the methods such as described in WO2010/035769, WO2010/107108, and WO2010/106812. Specifically, antibodies can be produced using genetic recombination techniques known to those skilled in the art, based on the sequence of the above-mentioned anti-IL-6 receptor antibody (see, for example, Borrebaeck CAK and Larrick JW, THERAPEUTIC MONOCLONAL ANTIBODIES, Published in the United Kingdom by MACMILLAN PUBLISHERS LTD, 1990). A recombinant antibody can be obtained by cloning a DNA encoding the antibody from a hybridoma or an antibody-producing cell such as an antibody-producing sensitized lymphocyte, inserting the DNA into an appropriate vector, and introducing the vector into a host (host cell) to produce the antibody.

Such antibodies can be isolated and purified using isolation and purification methods conventionally used for antibody purification, without limitation. For example, the antibodies can be isolated and purified by appropriately selecting and combining column chromatography, filtration, ultrafiltration, salting-out, solvent precipitation, solvent extraction, distillation, immunoprecipitation, SDS-polyacrylamide gel electrophoresis, isoelectric focusing, dialysis, recrystallization, and so on.

The antibodies used in the present invention may be conjugate antibodies that are bound to various molecules such as polyethylene glycol (PEG), radioactive substances, and toxins. Such conjugate antibodies can be obtained by chemically modifying the obtained antibodies. Methods for antibody modification have been already established in this field. Accordingly, the term "antibody" as used herein encompasses such conjugate antibodies.

"Antibodies" of the present invention include those that have been post-translationally modified. Post-translational modifications include, but are not limited to, modification of a heavy-chain or light-chain N-terminal glutamine or glutamic acid into a pyroglutamic acid by pyroglutamylation.

Herein, the term "pharmaceutical composition" (which can also be expressed as a suppressor, function-restoring promoter, function-restoring agent, therapeutic agent, and preventive agent) indicates a preparation in a form that allows the biological activity of the active ingredient contained therein to exert an effect, which does not contain any additional ingredient that is toxic to an unacceptable degree to the subject to which a formulation is administered. The pharmaceutical composition of the present invention may comprise more than one active ingredient, if that is necessary for its suppressive, function-restoring, therapeutic, or preventive purpose. Those with complementary activities that do not adversely affect each other are preferred. Such active ingredients are present in suitable combination in amounts that are effective for the intended purpose.

Pharmaceutical compositions of the present invention used for suppressive, function-restoring, therapeutic, or preventive purposes can be formulated to produce freeze-dried formulations or solution formulations by mixing, if necessary, with suitable pharmaceutically acceptable carriers, vehicles, and such. The suitable pharmaceutically acceptable carriers and vehicles include, for example, sterilized water, physiological saline, stabilizers, excipients, antioxidants (such as ascorbic acid), buffers (such as phosphate, citrate, histidine, and other organic acids), antiseptics, surfactants (such as PEG and Tween), chelating agents (such as EDTA), and binders. Other low-molecular-weight polypeptides, proteins such as serum albumin, gelatin, and immunoglobulins, amino acids such as glycine, glutamine, asparagine, glutamic acid, aspartic acid, methionine, arginine, and lysine, sugars and carbohydrates such as polysaccharides and monosaccharides, and sugar alcohols such as mannitol and sorbitol may also be contained. When preparing an aqueous solution for injection, physiological saline and isotonic solutions comprising glucose and other adjuvants such as D-sorbitol, D-mannose, D-mannitol, and sodium chloride may be used; and appropriate solubilizers such as alcohol (for example, ethanol), polyalcohols (such as propylene glycol and PEG), and nonionic surfactants (such as polysorbate 80, polysorbate 20, poloxamer 188, and HCO-50) may be used in combination. By mixing hyaluronidase into the formulation, a larger fluid volume can be administered subcutaneously (Expert Opin. Drug Deliv. 2007 Jul; 4(4): 427-40). Furthermore, syringes may be prefilled with the pharmaceutical composition of the present invention. Solution formulations can be prepared according to the method described in WO2011/090088.

If necessary, the pharmaceutical compositions of the present invention may be encapsulated in microcapsules (e.g., those made of hydroxymethylcellulose, gelatin, and poly(methylmetacrylate)), or incorporated into colloidal drug delivery systems (e.g., liposomes, albumin microspheres, microemulsion, nanoparticles, and nanocapsules) (see, for example, "Remington's Pharmaceutical Science 16th edition", Oslo Ed. (1980)). Methods for preparing the pharmaceutical agents as controlled-release pharmaceutical agents are also known, and such methods may be applied to the pharmaceutical compositions of the present invention (Langer et al., J. Biomed. Mater. Res. 15: 267-277 (1981); Langer, Chemtech. 12: 98-105 (1982); U.S. Patent No. 3,773,919; European Patent Application Publication No. EP 58,481; Sidman et al., Biopolymers 22: 547-556 (1983); and EP 133,988).

The pharmaceutical composition of the present invention can be administered to a patient via any appropriate route. For example, it can be administered to a patient intravenously by bolus injection or by continuous infusion, intramuscularly, intraabdominally, intracerebrospinally, transdermally, subcutaneously, intraarticularly, sublingually, intrasynovially, orally, by inhalation, locally, or externally, for a certain period of time. In one embodiment, administration of the pharmaceutical composition of the present invention is systemic administration, and shows an adhesion-suppressing effect at sites of surgical invasion in the whole body.

All prior art documents cited in the present specification are incorporated herein by reference.

### [Example]

Next, the present invention will be described in more detail by way of examples, but it is not limited thereto.

### [Effect of SA237 on the barrier function of BBB]

### (Experimental method)

### Production of an in vitro BBB model

In order to evaluate the barrier function of BBB, an *in vitro* BBB model prepared according to the method described in Examples 1-1, 1-2, 1-3, and 1-4 of International Publication No. WO2017/179375A1 was used. The prepared BBB model co-cultured at 37°C for five days was used for evaluating the barrier function. Fig. 1 shows a schematic diagram of the *in vitro* BBB model used in this study.

### Evaluation of barrier function

Changes over time in transepithelial/endothelial electrical resistance (TEER) values when IgGs purified from pooled sera from NMOSD patients or healthy subjects or SA237 were added from the produced *in vitro* BBB model's upper layer which is the endothelial cell side (corresponding to the vascular lumen side), the lower layer which is the astrocyte side (corresponding to the central nervous system side), or from both sides, were evaluated using CellZscope (CellSeed Inc., CSZ12001, CSZ24001), which is a cell tight junction real-time monitoring system.

### (Results)

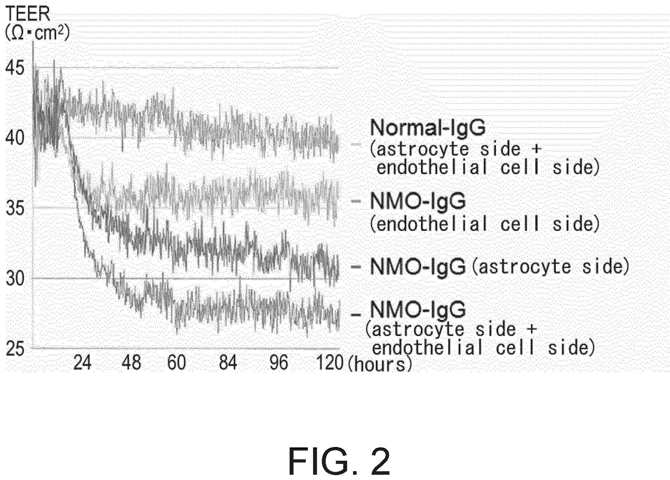

IgGs purified from NMOSD patients' pooled sera or healthy subjects' pooled sera were made to act from the endothelial cell side, which is the upper layer of the produced *in vitro* BBB model, the astrocyte side, which is the lower layer, or both sides, and the changes in TEER over time (five days) were evaluated using CellZscope (Fig. 2). As shown in Fig. 2, when IgGs purified from NMSOD patients' pooled sera (NMOSD-IgG) were made to act, TEER decreased as compared to the group in which IgGs purified from healthy subjects' pooled sera (Normal-IgG) were made to act. The decrease in TEER was observed approximately 24 hours after the addition of NMOSD-IgGs and persisted until at least 120 hours thereafter. In addition, a decrease in TEER was observed in all of the cases where NMOSD-IgGs were made to act from the astrocyte side and endothelial cell side, from the astrocyte side, and from the endothelial cell side, and the extent of TEER decrease was larger in this order. From these results, it was revealed that NMOSD-IgGs reduce the barrier function of BBB on both the endothelial cell side and the astrocyte side.

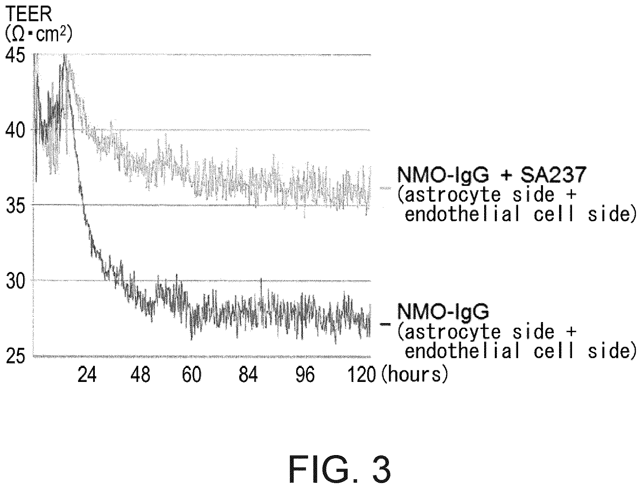

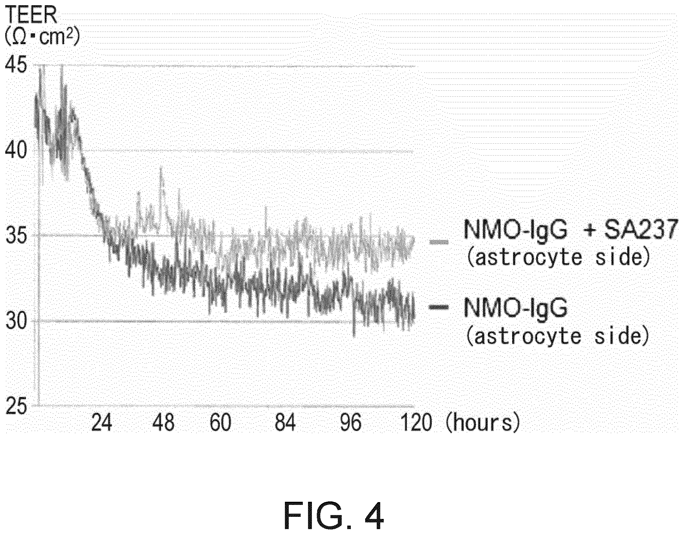

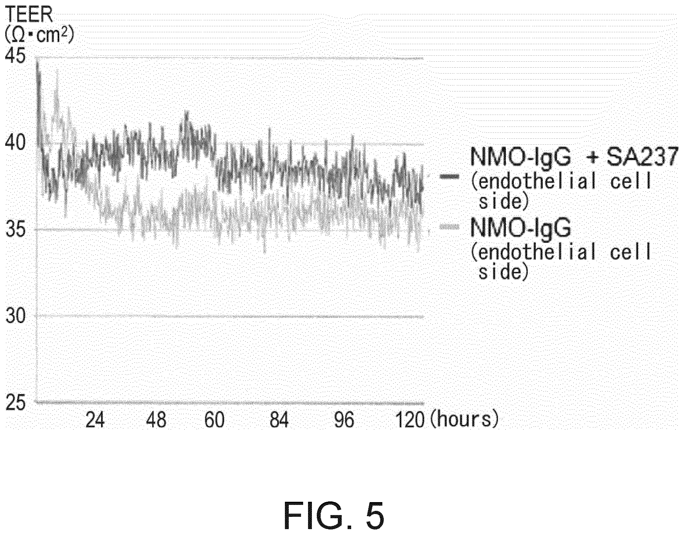

Next, the effect on TEER when SA237 was made to act at the same time as NMOSD-IgGs was evaluated (Figs. 3, 4, and 5). As shown in Fig. 3, when NMOSD-IgGs and SA237 (100 µg/mL) were made to act from the astrocyte side and the endothelial cell side, SA237 suppressed the TEER decrease caused by NMOSD-IgGs by about 30%. In addition, as shown in Figs. 4 and 5, SA237 similarly suppressed TEER decrease caused by NMOSD-IgGs by about 17% and about 9% when NMOSD-IgGs and SA237 (100 µg/mL) were made to act from each of the astrocyte side and the endothelial cell side, respectively. These results revealed that SA237 suppresses the decrease in BBB barrier function (more specifically, the disruption of the tight junctions of the blood-brain barrier) by NMOSD-IgGs at both the endothelial cell side and the astrocyte side.

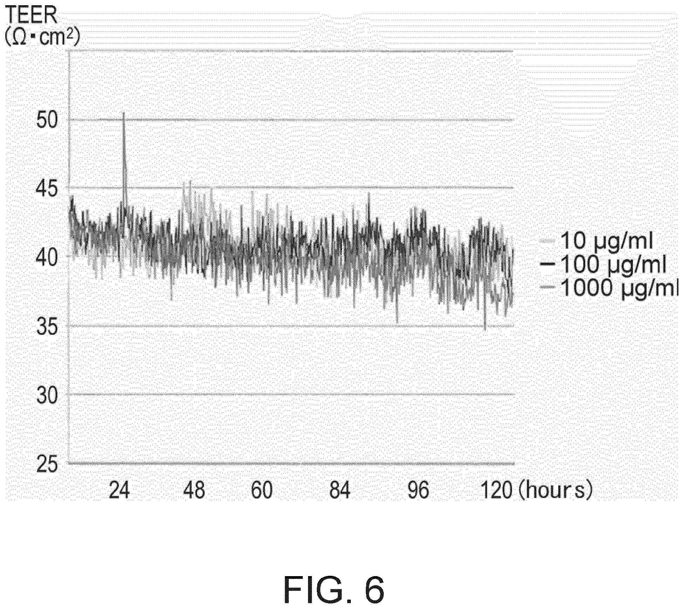

Finally, in order to evaluate whether SA237 itself can affect TEER, SA237 alone was made to act on the *in vitro* BBB model (Fig. 6). As shown in Fig. 6, when SA237 alone was made to act from the astrocyte side and the endothelial cell side, it did not affect TEER at any of the concentrations of 10, 100, and 1000 µg/mL. From the above results, it was revealed that SA237 itself does not affect the BBB barrier function.

### [Improving effect of SA237 on BBB with reduced barrier function]

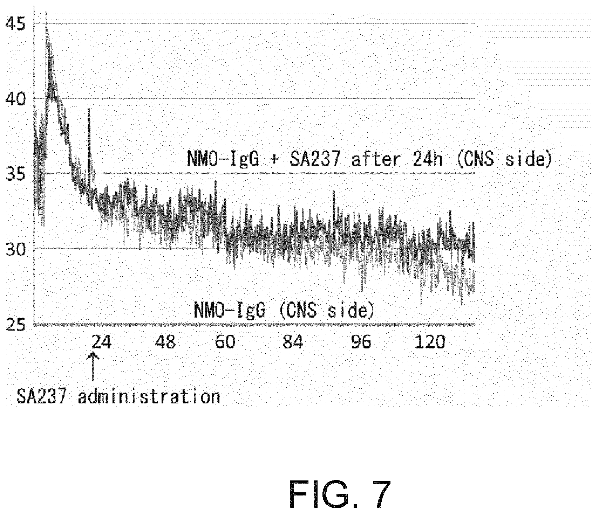

In the same manner as described above, NMOSD-IgGs were made to act on the same model as the *in vitro* BBB model used in the above Example, SA237 was added from after 24 hours or later, and the influence on TEER as measured by CellZscope was evaluated (Fig. 7). Both NMOSD-IgGs and SA237 were made to act from the astrocyte side (corresponding to the central nervous system side) of the *in vitro* BBB model. As shown in Fig. 7, TEER was significantly increased in the group in which SA237 was made to act 24 hours after NMOSD-IgGs were made to act, as compared with the group in which SA237 was not administered. From this result, it was revealed that SA237 restores the BBB barrier function that was reduced (more specifically, the tight junctions of the blood-brain barrier that had been disrupted) due to NMOSD-IgGs.

### [Effect of SA237 on BBB permeability to IgGs]

In the same manner as described above, IR-Dye-labeled NMOSD-IgGs were made to act from the endothelial cell side, which is the upper layer of the *in vitro* BBB model used in the above Examples, and then IgG permeability against BBB was assessed by measuring the amount of fluorescence of NMOSD-IgGs on the astrocyte side, which is the lower layer, using the Odyssey imaging system manufactured by LI-COR, and the effect of SA237 on this was evaluated. The amount of IgGs was evaluated by using fluorescently-labeled IgGs and measuring the amount of fluorescence with a fluorescence plate reader.

### [Effect of SA237 on leukocyte infiltration into BBB]

A membrane co-cultured with microvascular endothelial cells/pericytes/astrocytes was mounted on a dedicated chamber of a flow velocity load type BBB model (Takeshita Y et al., J Neurosci Methods 2014 Jul 30; 232: 165-172), and the lid was closed and sealed. The sealed chamber was placed in a chamber warmer set at 37°C, and PBMCs kept warm in a water tank were flowed into the chamber using a flow pump. By analyzing leukocytes that infiltrated to the outside of the membrane in the presence of NMOSD-IgGs at this time, the effect of NMOSD-IgGs on promoting leukocyte infiltration into the BBB and the BBB function related to leukocyte infiltration were evaluated, and the effect of SA237 thereon was assessed.

### (Experimental method)

### PBMC isolation

PBMCs were isolated from fresh heparinized blood of healthy subjects by density centrifugation using a Lymphocyte Separation Medium (Mediatech, Herndon, VA), which is a method known to those skilled in the art. PBMCs were resuspended to be 10 x 10⁶ cells in 30 ml TEM buffer (RPMI 1640 containing no phenol red, 1% bovine serum albumin, and 25 mM Hepes) and used in the assay within two hours after phlebotomy. PBMCs were stained with Calcein AM (Invitrogen) according to the manufacturer's protocol prior to perfusion into the chamber.

### Leukocyte infiltration assay

A leukocyte infiltration assay under flow velocity loading was performed using a 3D Flow Chamber Device (C.B.S. Scientific) (see Takeshita Y et al., J Neurosci Methods 2014 Jul 30; 232: 165-72). This system is composed of a 3D flow pump, a 3D flow chamber, and a 3D flow membrane. The 3D flow pump provides a wide range of programmable shear flows for up to eight flow devices (0.1-200 dyne/cm²). The 3D flow chamber (depth: 30 mm, width: 70 mm, height: 8 mm) has three separate reservoirs in which the 3D flow membrane fits totally. The 3D flow membrane is a membrane made of track-etched polycarbonate with a diameter of 8 mm and small pores of 3 µm. The membrane was coated with rat tail collagen I solution (50 µg/ml) (BD Bioscience, San Diego, CA) and placed in a 12-well plate. Human endothelial cells, human pericytes, and human astrocytes were multi-cultured in these wells at 33°C for two days using a medium for astrocytes. The cultured membrane was incubated at 37°C for one day and transferred to the chamber. PBMCs kept at 37°C were perfused into the chamber for 60 minutes under the conditions of a final concentration of 333,000 cells/ml and a shear stress of 1.5 dyne/cm² (flow velocity load). After perfusion of PBMCs, free cells were removed by flushing the chamber with PBS for five minutes while maintaining the same shear stress as in the assay. PBMCs that had passed through the membrane were collected from the bottom chamber. Cells that adhered to the abluminal side of the membrane and bottom chamber were removed by rapid washing using 0.5 mM EDTA. The number of recovered PBMC cells was counted using a hemocytometer. Subsequently, the collected cells were fixed with 1% PFA for 10 minutes, washed with PBS + 0.1 mM EDTA, and then blocked with mouse IgG. The cells were labelled with anti-human CD45 efluor 450, anti-human CD8a APC-efluor 780 (eBiosciences, San Diego, CA), anti-human CD3 Alexa Fluor 647 (Biolegend, San Diego, CA), anti-human CD19 BV711 and anti-human CD4 PE-CF594 (BD Biosciences). Data was obtained by flow cytometry using BD FACSCanto II (BD Biosciences), and the cell numbers of CD4⁺ cells, CD8⁺ cells, and CD19⁺ cells were analyzed using Flowjo 10.4.1 (Treestar, Ashland, OR).

### (Results)

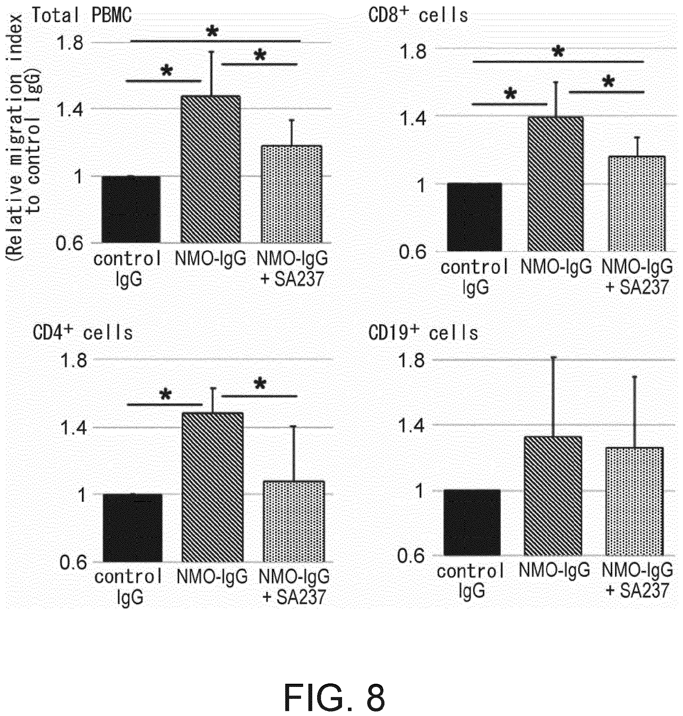

The number of total PBMCs, CD4⁺ cells, and CD8⁺ cells that had passed to the outside of the membrane increased in the group treated with NMOSD-IgG compared to the group treated with control IgG. On the other hand, the number of total PBMCs, CD4⁺ cells, and CD8⁺ cells that had passed to the outside of the membrane was significantly reduced in the group in which NMOSD-IgG and SA237 were made to act as compared with the group in which NMOSD-IgG alone was made to act (Fig. 8). From these results, it was revealed that SA237 suppresses the translocation promoted by NMSOD-IgG of total PBMCs, CD4⁺ cells, and CD8⁺ cells to the outside of the membrane.

### [Industrial Applicability]

The suppressors of the present invention provide a novel means capable of exerting a suppressive effect on reduced blood-brain barrier functions such as disruption of tight junctions of the blood-brain barrier, infiltration of leucocytes into the CNS, and permeation of IgGs in a patient's blood into the CNS. The therapeutic agents of the present invention provide a novel means capable of suppressing the reduction of blood-brain barrier functions, and thus treating various diseases involving a high concentration of IL-6 in the cerebrospinal fluid and reduced blood-brain barrier functions.

## Claims

1. A suppressor of the reduction of blood-brain barrier function, comprising an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6.

2. The suppressor according to claim 1, wherein the reduction of blood-brain barrier function is a disruption of a tight junction of the blood-brain barrier.

3. The suppressor according to claim 1, wherein the reduction of blood-brain barrier function is infiltration of leukocytes into the CNS.

4. The suppressor according to claim 1, wherein the reduction of blood-brain barrier function is permeation of IgGs in a patient's blood into the CNS.

5. The suppressor according to any one of claims 1 to 4, which suppresses the reduction of blood-brain barrier function, the disruption of a tight junction of the blood-brain barrier, infiltration of leukocytes into the CNS, or permeation of IgGs in a patient's blood into the CNS in a patient with neuromyelitis optica spectrum disorder, neuro-Behcet disease, neurosarcoidosis, central nervous system lupus (neuropsychiatric lupus), autoimmune encephalitis, or Vogt-Koyanagi-Harada disease.

6. A therapeutic agent for acute neuromyelitis optica spectrum disorder, wherein the therapeutic agent comprises an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6.

7. An agent for restoring blood-brain barrier function, wherein the agent comprises an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6.

8. A therapeutic agent for neuromyelitis optica spectrum disorder, neuro-Behcet disease, neurosarcoidosis, central nervous system lupus (neuropsychiatric lupus), autoimmune encephalitis, or Vogt-Koyanagi-Harada disease, wherein the therapeutic agent comprises an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6, and wherein the therapeutic agent suppresses the reduction of blood-brain barrier function and/or restores a reduced blood-brain barrier function.

9. The therapeutic agent according to claim 8, wherein the reduction of blood brain barrier function is a disruption of a tight junction of the blood-brain barrier.

10. The therapeutic agent according to claim 8, wherein the reduction of blood-brain barrier function is infiltration of leukocytes into the CNS.

11. The therapeutic agent according to claim 8, wherein the reduction of blood-brain barrier function is permeation of IgGs in a patient's blood into the CNS.

12. A therapeutic agent for neurosarcoidosis, central nervous system lupus (neuropsychiatric lupus), or Vogt-Koyanagi-Harada disease, wherein the therapeutic agent comprises an antibody comprising a heavy chain variable region comprising CDR1 having the sequence of SEQ ID NO: 1, CDR2 having the sequence of SEQ ID NO: 2, and CDR3 having the sequence of SEQ ID NO: 3, and a light chain variable region comprising CDR1 having the sequence of SEQ ID NO: 4, CDR2 having the sequence of SEQ ID NO: 5, and CDR3 having the sequence of SEQ ID NO: 6.

13. The suppressor or therapeutic agent according to any one of claims 1 to 12, wherein the antibody comprises an antibody comprising the light chain variable region of SEQ ID NO: 7 and the heavy chain variable region of SEQ ID NO: 8.

14. The suppressor or therapeutic agent according to any one of claims 1 to 13, wherein the antibody comprises an antibody comprising the light chain of SEQ ID NO: 9 and the heavy chain of SEQ ID NO: 10.
